# EUROPEAN PATENT APPLICATION

(11) **EP 4 205 772 A1**
(43) Date of publication of application: **05.07.2023**
(21) Application number: 21864042.3
(22) Date of filing: 05.08.2021
(51) Int. Cl.: A61L 2/20, A61L 9/04, A61L 9/20

(54) **STERILIZATION TREATMENT METHOD AND STERILIZATION TREATMENT DEVICE**

(30) Priority: 07.09.2020 JP 2020149898
(71) Applicant: Ushio Denki Kabushiki Kaisha, Tokyo 100-8150 (JP)
(72) Inventor: NAITO, Keisuke, Tokyo 100-8150 (JP)
(74) Representative: Tomerius, Isabel
(86) International application number: PCT/JP2021/029051
(87) International publication number: WO 2022/049984

(57) **Abstract**

Provided are a sterilization treatment method and a sterilization treatment device that each use ozone and produce an improved sterilizing effect in a short time. The sterilization treatment method is a method for sterilization treatment by ozone in a treatment space defined and includes introducing ozone into the treatment space in an environment in which an amphiphilic substance is present.

## Description

### TECHNICAL FIELD

The present invention relates to a sterilization treatment method and a sterilization treatment device, and particularly relates to a sterilization treatment method and a sterilization treatment device that each use ozone.

### BACKGROUND ART

A method for sterilization using ozone is known as a method for killing germs present in a space. For example, Patent Document 1 below discloses a method for sterilization treatment performed by supplying an ozone gas into a chamber.

### PRIOR ART DOCUMENT

### PATENT DOCUMENT

Patent Document 1: JP-A-2017-164260

### SUMMARY OF THE INVENTION

### PROBLEMS TO BE SOLVED BY THE INVENTION

Ozone has strong oxidizing power and thus is used to kill various germs and inactivate viruses. However, among germs, bacteria producing spores (hereinafter referred to as "spore-forming bacteria"), such as bacteria that belong to Bacillus like Bacillus subtilis, Bacillus cereus, and anthrax and bacteria that belong to Clostridium like Clostridium botulinum and Clostridium tetani, have outer shells that cannot be infiltrated or broken by ozone. Such bacteria cannot be killed only by being exposed to ozone.

Hence, methods for killing spore-forming bacteria by hydroxyl radicals have been proposed. The hydroxyl radical, among other active oxygen species, has very strong oxidizing power and is generated by ozone dissolved in water.

Hydroxyl radicals are highly reactive among active oxygen species and thus cannot be present in air for a long time and immediately disappear after being generated. As a result, to allow spore-forming bacteria to be killed by hydroxyl radicals, the hydroxyl radical needs to be generated near the spore-forming bacteria.

A method for achieving this is the method for sterilization treatment, disclosed in Patent Document 1, by which water in mist form is sprayed in the chamber and a hydroxyl radical is generated by a reaction of the water that remains on a surface of the bacteria with ozone.

Unfortunately, the surface of the bacteria, even under an environment of high humidity, repels moisture that comes into contact with the surface due to factors such as a chemical composition and the presence of an oil content, and the water is difficult to remain on the surface. Hence, even when the ozone reaches the surface of the bacteria, the hydroxyl radical is hardly generated.

It is considered that a start of ozone generation is waited for a period of time until adequate water adheres to the surface of the bacteria under a high humidity environment so that the water satisfactorily remains on the surface of the bacteria to cause a hydroxyl radical to be generated near the bacteria. However, in order for the sterilization treatment method to produce an expected sterilizing effect, a treatment space needs to be kept humidified for a long time and be maintained at high humidity to cause the water to reliably remain on the surface of the bacteria.

In view of the above problem, it is an object of the present invention to provide a sterilization treatment method and a sterilization treatment device that each use ozone and produce an improved sterilizing effect in a short time.

### MEANS FOR SOLVING THE PROBLEMS

A sterilization treatment method according to the present invention is a method for sterilization treatment by ozone in a treatment space defined, and includes introducing ozone into the treatment space in an environment in which an amphiphilic substance is present.

Ozone present in water generates a hydroxy radical (·OH) by a process of formulas (1) to (2) below. The formula (1) shows a process of generation of the hydroperoxyl radical (·HO₂) and the superoxide anion (O₂⁻) through a reaction of the ozone with the hydroxide ion (OH⁻) present in the water. The formula (2) shows a process of generation of oxygen and the hydroxy radical through a reaction of the ozone with the hydroperoxyl radical. The only two typical reactions are described herein, although many reactions other than the formulas (1) and (2) can occur serially.

O₃ + OH⁻ → ·HO₂ + O₂⁻ ···· (1)

O₃ + ·HO₂ → 2O₂ + ·OH ···· (2)

The amphiphilic substance is a substance that possesses both a hydrophilic group compatible with moisture and a lyophilic group (a hydrophobic group) compatible with an oil content. When the amphiphilic substance comes into contact with an oil content present on a surface of the bacteria or a part of the bacteria that has a chemical composition compatible with the lyophilic group, the amphiphilic substance remains on the surface of the bacteria such that the lyophilic group faces the surface of the bacteria and the hydrophilic group faces a side opposite the surface of the bacteria. Consequently, the hydrophilic groups of the amphiphilic substance appear at places of the outer surface of the bacteria, thus forming an area compatible with water.

Thus, by the method described above, the ozone is introduced in a condition in which such an area readily compatible with water is formed. This facilitates the generation of a hydroxyl radical on the surface of or near the bacteria.

Since the generated hydroxyl radical is generated on the surface of or near the bacteria, the hydroxyl radical is apt to come into contact with the bacteria before disappearing through a reaction with a substance in the air. In other words, the method described above enables both sterilization treatment against ordinary bacteria with increased reliability and sterilization treatment against bacteria, such as spore-forming bacteria, that has an outer shell and high resistance.

The sterilization treatment method may be a method including generating ozone after spraying the amphiphilic substance diluted with water in the treatment space.

The amphiphilic substance is a stable substance. Thus, after an amphiphilic substance that is diluted with water is sprayed in the treatment space, the amphiphilic substance does not gradually react or decompose but is able to maintain a state for a period of time in which the amphiphilic substance is suspended or adheres to the bacteria or the like in the treatment space. On the other hand, ozone is a very unstable substance that gradually decomposes and becomes oxygen at room temperature. Thus, even in a closed space, a concentration of the ozone in the treatment space gradually decreases if the ozone is not constantly generated.

Thus, with the above method, the bacteria in the treatment space are in a state in which the water satisfactorily remains on the surface of the bacteria when the ozone is generated in the treatment space. As a result, the ozone reacts with the water that remains on the surface of the bacteria, and this further facilitates the generation of a hydroxyl radical and enables sterilization treatment against bacteria, such as spore-forming bacteria, that has an outer shell and high resistance with increased reliability.

The sterilization treatment method may be a method including generating ozone after spraying the amphiphilic substance in the treatment space.

For instance, if the treatment space is a space such as a bathroom in which humidity is present to a certain extent, the sterilization treatment method may be a method including spraying only the amphiphilic substance such that the humidity present in the space readily remains on the surface of the bacteria.

In the sterilization treatment method, the amphiphilic substance may be a substance that does not have sterilizing action on spore-forming bacteria.

In the sterilization treatment method, the amphiphilic substance may be alcohol or a surface-active agent.

Preferably, in the sterilization treatment method, the amphiphilic substance is ethanol that is diluted with water such that a concentration of the ethanol ranges from 3% by volume to 60% by volume inclusive.

Preferably, in the sterilization treatment method, the amphiphilic substance is polyoxyethylene sorbitan monooleate that is diluted with water such that a concentration of the polyoxyethylene sorbitan monooleate ranges from 0.5 g/L to 5 g/L inclusive.

If the concentration of the amphiphilic substance is too low, the amphiphilic substance adhering to bacteria present in the treatment space might be inadequate. If the concentration is too high, alcohol such as ethanol might gradually vaporize due to high volatility even while the space is humidified, for example, and might completely vaporize before ozone reaches the bacteria. A surface-active agent such as polyoxyethylene sorbitan monooleate might be compatible with an oil content everywhere in the treatment space, and it could take time to ventilate and clean the space after sterilization treatment.

Hence, it is preferred that the amphiphilic substances are diluted with water to reach the concentrations in the respective ranges described above.

In the sterilization treatment method, the ozone introduced into the treatment space may be generated by an ozone generator that includes a discharge lamp to emit ultraviolet light having a main emission wavelength in a range from 100 nm to 240 nm inclusive.

In this specification, the "main emission wavelength" indicates, when a wavelength range Z(λ) covering ±10 nm of a wavelength λ is defined in an emission spectrum, a wavelength λi in the wavelength range Z(λi) at which an integrated intensity accounts for 40% or more of a total integrated intensity of the emission spectrum. For a light source, such as an excimer lamp in which a luminescent gas containing KrCl, KrBr, and ArF is sealed, that has an extremely narrow half-value width and displays light intensities only at specific wavelengths, a wavelength at which the relative intensity is highest (a main peak wavelength) may be, in most cases, regarded as the main emission wavelength.

When oxygen is irradiated with ultraviolet light at wavelengths of the light absorption spectrum of oxygen in a range from 100 nm to 240 nm inclusive, reactions shown in formulas (3) to (4) below proceed and ozone is generated. In the formula (3), hv represents absorption of the ultraviolet light. In the formulas (3) and (4), both O('D) and O(³P) are comprehensively represented by "O·". In the formula (3) in particular, whether or not O('D) is generated depends on the wavelength of the ultraviolet light with which the oxygen is irradiated.

O₂ + hv → O· + O· ···· (3)

O· + O₂ → O₃ ···· (4)

A sterilization treatment device according to the present invention includes: a humidifier that includes a reservoir to store an amphiphilic substance diluted with water, and a jet port to emit the amphiphilic substance diluted with the water in mist form, the amphiphilic substance flowing into the jet port from the reservoir; and an ozone generator that includes a discharge lamp to emit ultraviolet light having a main emission wavelength in a range from 100 nm to 240 nm inclusive when an electrode is supplied with electric power, a gas inlet to take in air, and a gas outlet to discharge an ozone-containing gas that is generated when air taken in through the gas inlet is irradiated with the ultraviolet light from the discharge lamp.

The configuration above is designed to create a treatment space in which an amphiphilic substance is present, take in oxygen, and introduce ozone generated from the intake oxygen into the space in which the amphiphilic substance is present.

### EFFECT OF THE INVENTION

According to the present invention, it is possible to realize a sterilization treatment method and a sterilization treatment device that each use ozone and produce an improved sterilizing effect in a short time.

### BRIEF DESCRIPTION OF THE DRAWINGS

Fig. 1 is an overall side view schematically showing an embodiment of an environment for sterilization treatment.
Fig. 2A is a cross-sectional side view of a humidifier.
Fig. 2B is a cross-sectional view of an ozone generator.
Fig. 3 is an overall side view schematically showing an embodiment of an environment for sterilization treatment.
Fig. 4 is an overall side view showing an object to be treated being disposed in a box.
Fig. 5 is an overall side view showing a condition in which an amphiphilic substance in mist form is sprayed by a humidifier.
Fig. 6 is an overall side view showing a condition in which an ozone generator takes in air and discharges an ozone-containing gas.
Fig. 7 is an overall side view showing a condition in which an inside of a box is ventilated.
Fig. 8 is an overall side view showing an object to be treated being removed from a box.
Fig. 9 is a graph showing results of an experiment on sterilization treatment by ozone.
Fig. 10 is a graph showing results of an experiment on sterilization treatment by ozone.

### MODE FOR CARRYING OUT THE INVENTION

A sterilization treatment method and a sterilization treatment device according to the present invention will be described below with reference to the drawings. It is to be noted that the drawings referred to below regarding the sterilization treatment device are all schematic illustrations and dimensional ratios and numbers of parts on the drawings do not necessarily match the actual dimensional ratios and numbers of parts.

### [Sterilization treatment device]

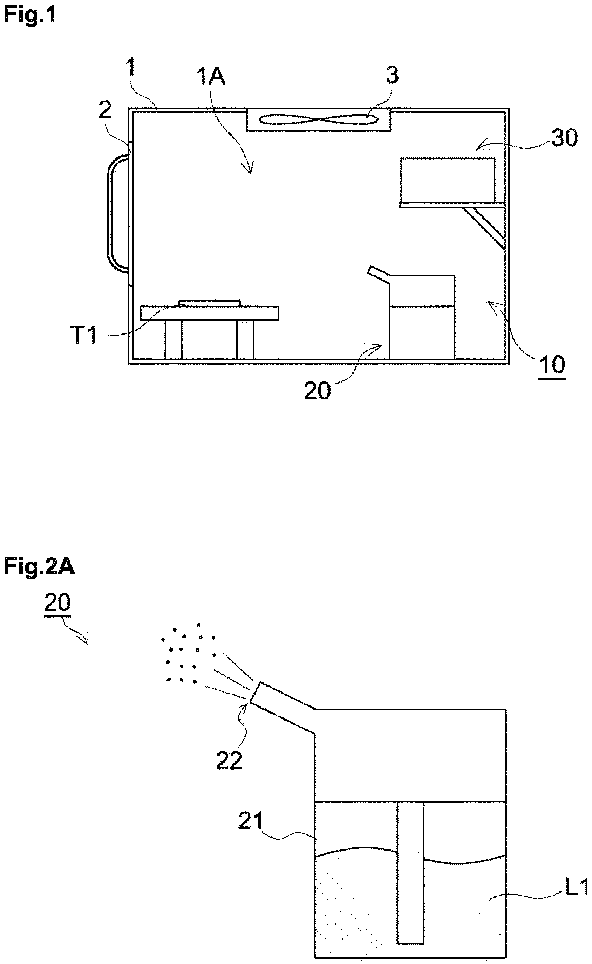

Fig. 1 is an overall side view schematically showing an embodiment of an environment for sterilization treatment. As shown in Fig. 1, a box 1 includes a door 2 to take an object to be treated in and out of and a ventilating fan 3 to ventilate a treatment space 1A. A sterilization treatment device 10 includes a humidifier 20 and an ozone generator 30 and is disposed inside the treatment space 1A defined by the box 1.

The treatment space 1A is hereinafter described as a space defined by the box 1. However, the sterilization treatment device 10 can be used in a space defined by a range in which humidity can be adjusted by the humidifier 20 and that can be reached by ozone without being decomposed. For instance, the sterilization treatment device may be used for sterilization treatment in a room such as a meeting room and a hospital room.

The treatment space 1A inside the box 1 may not be a fully enclosed space. Further, the sterilization treatment device 10 can be used, for example, for sterilization treatment of food, a medical device, plant seed, or the like disposed in the box 1 and for sterilization treatment of indoor equipment such as a table, a sofa, and a curtain disposed in a room regarded as the treatment space 1A.

As shown in Fig. 1, the present embodiment is described as an example of sterilization treatment against bacteria adhering to an object to be treated T1 placed in the box 1.

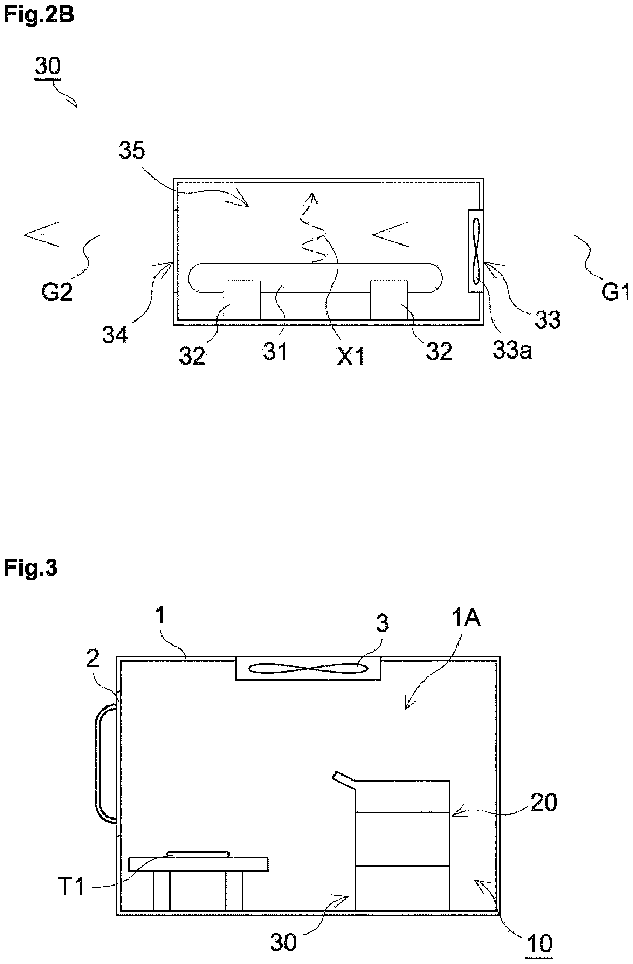

Fig. 2A is a cross-sectional side view of the humidifier 20. As shown in Fig. 2, the humidifier 20 includes a reservoir 21 to store a chemical solution L1 that is ethanol diluted with water and a jet port 22 to emit the chemical solution L1 in mist form. An ultrasonic humidifier, for example, can be used as the humidifier 20, which may alternatively be a generally available humidifier for household use.

Ethanol is an amphiphilic substance that has both a hydrophilic hydroxyl group and a hydrophobic alkyl group. In the present embodiment, the chemical solution L1 is a liquid diluted with distilled water such that a concentration of the ethanol is 50% by volume. Ethanol is not harmful if ethanol ingested by a human is small in quantity. If ethanol is directly sprayed on plant seeds, the ethanol is not likely to kill the seeds.

Preferably, from the viewpoint of effects, volatility, and other properties exhibited by the amphiphilic, the chemical solution L1 is such that the ethanol concentration ranges from 3% by volume to 60% by volume inclusive. The chemical solution L1 may be any liquid with proviso that an amphiphilic substance possessing both hydrophilic and hydrophobic characteristics is diluted with water. A specific amphiphilic substance that can be used is alcohol or a surface-active agent. More specifically, examples of the alcohol include ethanol, isopropyl alcohol, acetone, ethylene glycol, and diethyl ether, and examples of the surface-active agents include nonionic surface-active agents, cationic surface active agents, and anionic surface active agents. Preferably, the alcohol and the surface-active agent are ethanol and polyoxyethylene sorbitan monooleate (also referred to as "polysorbate 80" or "tween 80"), respectively.

Even if any of ethanol and polyoxyethylene sorbitan monooleate is directly sprayed on spore-forming bacteria to have the substance adhere to the bacteria, any of these substances cannot break an outer shell of the spore-forming bacteria and cannot break protein and DNA by infiltrating into the spore-forming bacteria. In other words, ethanol and polyoxyethylene sorbitan monooleate are substances that do not have sterilizing action on spore-forming bacteria.

If polyoxyethylene sorbitan monooleate is used, the chemical solution L1 is preferably made such that a concentration of the polyoxyethylene sorbitan monooleate ranges from 0.5 g/L to 5 g/L inclusive, from the viewpoint of effects exhibited by the amphiphilic, cleaning inside the treatment space 1A, and the like. The chemical solution L1 may contain a substance besides water and the amphiphilic substance.

As shown in Fig. 2A, the jet port 22 makes the chemical solution L1 stored in the reservoir 21 a mist form and emits the solution into the box 1.

Fig. 2B is a cross-sectional view of the ozone generator 30. The ozone generator 30, as shown in Fig. 2B, includes a discharge lamp 31, a pair of electrodes 32 that also serve as a support base for the discharge lamp 31, a gas inlet 33, a gas outlet 34, and a flow path 35 through which the gas inlet 33 and the gas outlet 34 communicate.

In the present embodiment, the discharge lamp 31 is an excimer lamp in which xenon gas is sealed as a light-emitting gas in a tubular body. The discharge lamp emits ultraviolet light having a main emission wavelength of 172 nm by applying a voltage between the electrodes 32.

The discharge lamp 31 may be any excimer lamp that emits light having a main emission wavelength other than 172 nm, with proviso that the excimer lamp emits light at wavelengths in a range from 100 nm to 240 nm inclusive.

The excimer lamp may be an excimer lamp other than the excimer lamp, which includes, as shown in Fig. 2B, the electrodes 32 that are separated from each other along a tube axis of the discharge lamp 31 and that double as a support base. The ozone generator may have, for example, an excimer lamp that includes a cylindrical tubular body and electrodes opposed to each other in a radial direction or an excimer lamp that has what is called a flat tube shape and electrodes opposed to each other through a flat tubular body. A cross section of the excimer lamp cut along a plane orthogonal to a tube axis of the flat tubular body is rectangular.

The discharge lamp 31 may include, for example, a lamp such as a low-pressure mercury lamp rather than the excimer lamp.

The ozone generator 30 may be, for example, a discharge type ozonizer that is not based on light irradiation technology. Further, the ozone generator may be configured to supply ozone simply from a cylinder.

As shown in Fig. 2B, air G1 that is taken into the flow path 35 by an air intake fan 33a provided for the gas inlet 33 flows toward the gas outlet 34 while being exposed to ultraviolet light X1 emitted from the discharge lamp 31. In the present embodiment, the air G1 is air staying inside the box 1. However, the air may be air introduced from outside the box 1.

When the air G1 taken in through the gas inlet 33 flows in the flow path 35, the air G1 is irradiated with the ultraviolet light X1 emitted from the discharge lamp 31, and ozone is generated from oxygen contained in the air G1 through a process of the formulas (3) to (4) described above.

The air G1 flowing in the flow path 35 contains the ozone generated by the ultraviolet light X1 and is discharged as an ozone-containing gas G2 from the gas outlet 34.

In Fig. 2B, the ozone generator 30 includes the air intake fan 33a to take in the air G1 from the gas inlet 33, but may include an air-sending mechanism like the air intake fan 33a and so forth.

Fig. 3 is an overall side view schematically showing an embodiment of an environment for sterilization treatment apart from Fig. 1. As shown in Fig. 3, the humidifier 20 and the ozone generator 30 may be integrated together. A configuration in which the humidifier 20 and the ozone generator 30 are integrated together makes it possible to decrease a disposition space and unify a power supply system and suchlike. When the humidifier 20 and the ozone generator 30 are independent devices, these devices can be disposed at optimum places for sterilization treatment in response to conditions such as an extent of the treatment space 1A, and a shape of and a position of an object to be sterilized in the treatment space 1A.

### [Sterilization treatment method]

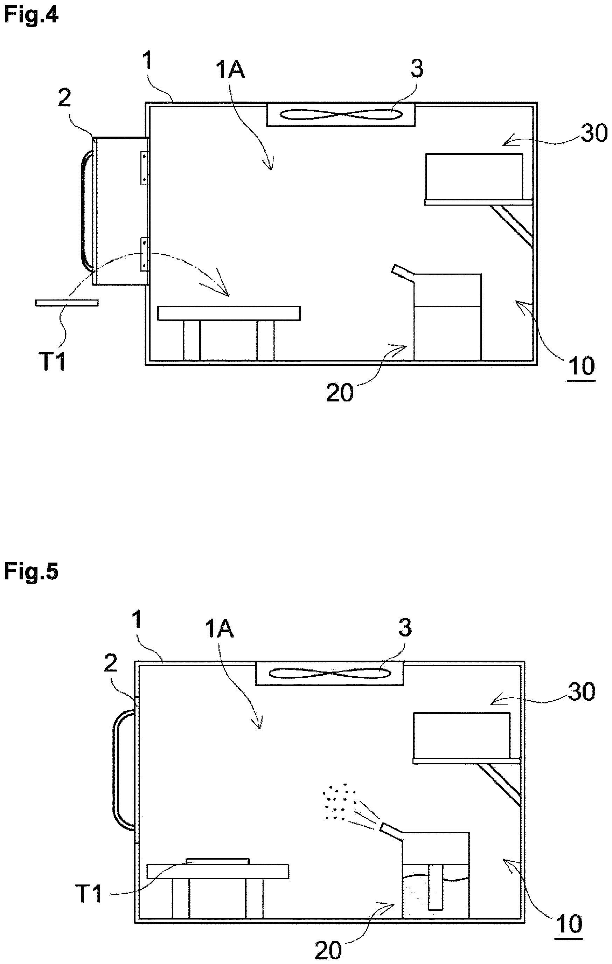

Next, a sterilization treatment method using the sterilization treatment device 10 is described. Fig. 4 is an overall side view showing the object to be treated T1 being disposed in the box 1. First, as shown in Fig. 4, the object to be treated T1 is disposed in the box 1 (step S1).

Fig. 5 is an overall side view showing a condition in which an amphiphilic substance in mist form is sprayed by the humidifier 20. As shown in Fig. 5, after step S1, the door 2 of the box 1 is closed, and the humidifier 20 starts an operation (step S2).

After step S2, when humidity inside the box 1 reaches 80% RH, the humidifier 20 stops the operation (step S3). If the amphiphilic substance is satisfactorily sprayed and it can be determined that water containing the amphiphilic substance in mist form has adhered to a surface of bacteria (especially spore-forming bacteria) adhering to the object to be treated T1, the humidifier 20 may stop the operation before the humidity reaches 80% RH.

If the humidity in the treatment space 1A is adjusted by the humidifier 20, it is preferred that the humidity is adjusted in a range from 60% RH to 95% RH, although desired humidity differs depending on a situation such as whether an inside of the box 1 is sterilized or a room is sterilized. When the humidity is too low, water can adhere to the bacteria only to a limited extent, and satisfactory sterilizing effect cannot be produced. When the humidity is too high, especially in a case of sterilization treatment of a room, books or other paper goods may be deformed or damaged by the humidity and electrical appliances or such products may break down due to condensation.

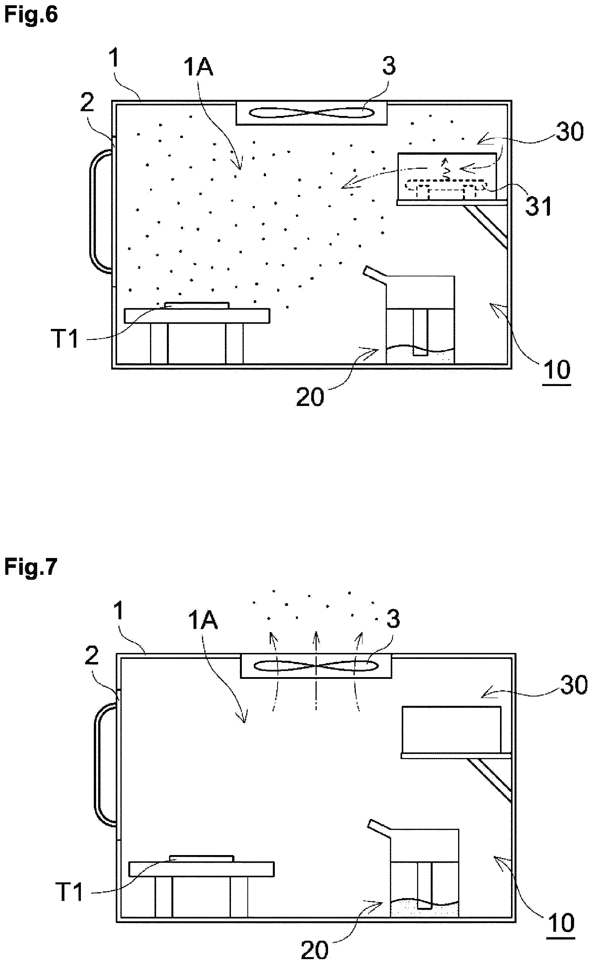

Fig. 6 is an overall side view showing a condition in which the ozone generator 30 takes in the air G1 and discharges the ozone-containing gas G2. In Fig. 6, the discharge lamp 31 and the electrodes 32 that are actually invisible are indicated by broken lines for convenience of description. After step S3, as shown in Fig. 6, the ozone generator 30 starts an operation (step S4).

When the ozone generator 30 starts the operation, the discharge lamp 31 is lit, the air G1 is taken into the flow path 35 by the air intake fan 33a, and the ozone-containing gas G2 containing ozone is released from the gas outlet 34.

Step S4 may be simultaneous with step S2, in other words, the humidifier 20 and the ozone generator 30 may start the operations simultaneously. In this case, step S3 is not executed. If before execution of step S2, the box 1 is already in an environment in which the amphiphilic substance is present in the box 1, steps S2 and S3 may be omitted.

Fig. 7 is an overall side view showing a condition in which the inside of the box 1 is ventilated. As shown in Fig. 7, after step S4, the ventilating fan 3 operates to ventilate the box 1 until concentrations of the ozone and the ethanol in the box 1 satisfactorily decrease (step S5).

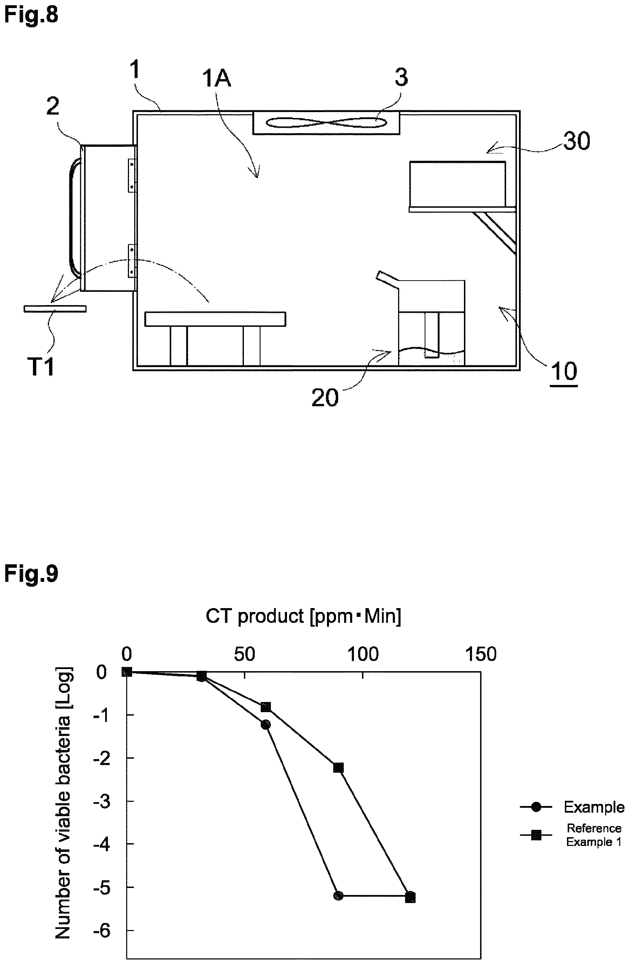

Fig. 8 is an overall side view showing the object to be treated T1 being removed from the box 1. After step S6, as shown in Fig. 8, the object to be treated T1 is removed from the box 1.

After a process of the steps above is executed, sterilization treatment of the object to be treated T1 is completed.

By the method described above, the amphiphilic substance present in the box 1 readily remains on the surface of the bacteria. Then, the water that remains on the surface of the bacteria reacts with the ozone, and a hydroxyl radical is generated on the surface of or near the bacteria. Thus, the generated hydroxyl radical is apt to come into contact with the bacteria before disappearing through a reaction with a substance in the air. This enables sterilization against the bacteria with increased reliability. The method enables sterilization treatment against spore-forming bacteria with increased reliability by breaking the outer shell of the spore-forming bacteria.

### [Experiment 1]

According to the method described above, an experiment was conducted using a biological indicator to compare sterilizing effects on spore-forming bacteria between varied concentrations of ethanol.

The biological indicator used in this experiment 1 was Biological Indicator H3726T made by Cosmo Bio Co., Ltd. for vaporized hydrogen peroxide sterilization.

The biological indicator is an indicator used to evaluate sterilizing effects. The biological indicator includes a strip of filter paper carrying spore-forming bacteria and a water-repellent paper bag in which the strip of the filter paper is sealed. The filter paper that has been treated inside the biological indicator is put in a dedicated culture medium and is incubated for a predetermined time. Then, if a change in color of the culture medium caused by a reaction with the remaining bacteria does not occur, the sterilizing effect of the treatment is evaluated as satisfactory.

The biological indicators were set, respectively, in a condition in which the filter paper carrying spore-forming bacteria is put in the water-repellent paper bag and in a condition in which the filter paper carrying spore-forming bacteria is taken out of the paper bag, and the experiment was conducted to compare results.

The treatment space 1A was defined by a box with a volume of 110 L.

After the biological indicator was placed in the box, the box was humidified until humidity inside the box 1 reached 80% RH to generate ozone.

A concentration of the ozone was adjusted to 500 ppm, and concentration-time (CT) products (ozone concentration [ppm] × time [Min]) were 0 ppm·Min (0 Min), 30000 ppm·Min (60 Min), 45000 ppm·Min (90 Min), 60000 ppm·Min (120 Min).

With ethanol diluted with distilled water, the chemical solutions L1 were prepared such that respective concentrations of the ethanol were 1% by volume, 2.5% by volume, 3% by volume, 5% by volume, 10% by volume, 20% by volume, 40% by volume, 50% by volume (Example), 60% by volume, and 70% by volume. In Tables 1 and 2 below, the concentrations are expressed briefly in units of "%".

In Reference Example 1, an experiment was conducted for a case in which the inside of the box was humidified with tap water.

In Reference Example 2, an experiment was conducted for a case in which without humidification, distilled water was directly dropped on the biological indicator. For the case with the paper bag, distilled water was dropped on an outer surface of the paper bag. For the case without the paper bag, distilled water was dropped on the content taken out of the paper bag.

Two trials were conducted for each, and results were determined as "A" when the color of the culture medium did not change in both two trials, were determined as "B" when the color of the culture medium changed in only one of the two trials, and were determined as "C" when the color of the culture medium changed in both two trials.

### (Results)

Table 1 below shows results of the experiment conducted in the condition in which the filter paper carrying spore-forming bacteria was taken out of the paper bag, whereas Table 2 shows results of the experiment conducted in the condition in which the filter paper carrying spore-forming bacteria was put in the paper bag.

**[Table 1]**

| CT product | Ethanol humidification | | | | | | | | | | Reference Example 1 | Reference Example 2 |
|---|---|---|---|---|---|---|---|---|---|---|---|---|
| | 1% | 2.5% | 3% | 5% | 10% | 20% | 40% | 50% | 60% | 70% | | |
| 0 | C | C | C | C | C | C | C | C | C | C | C | C |
| 30000 | C | C | C | A | A | A | A | A | A | A | C | A |
| 45000 | C | C | A | A | A | A | A | A | A | A | C | A |
| 60000 | C | C | A | A | A | A | A | A | A | A | C | A |

**[Table 2]**

| CT product | Ethanol humidification | | | | | | | | | | Reference Example 1 | Reference Example 2 |
|---|---|---|---|---|---|---|---|---|---|---|---|---|
| | 1% | 2.5% | 3% | 5% | 10% | 20% | 40% | 50% | 60% | 70% | | |
| 0 | C | C | C | C | C | C | C | C | C | C | C | C |
| 30000 | C | C | C | B | B | B | B | B | B | B | C | C |
| 45000 | C | C | A | A | A | A | A | A | A | A | C | C |
| 60000 | C | C | A | A | A | A | A | A | A | A | C | C |

When the ethanol concentrations of the chemical solutions L1 were in a range from 3% by volume to 70% by volume inclusive, sterilizing effects on the spore-forming bacteria were observed.

For the condition with the paper bag, even the distilled water directly dropped on the paper bag did not reach the filter paper due to water repellency of the paper bag. This disabled sterilization treatment against the spore-forming bacteria. In other words, in the case of humidification by the ethanol, due to an amphiphilic effect of the ethanol, moisture passed through the paper bag and reached the spore-forming bacteria. As a result, sterilization treatment was performed.

Ethanol exerts sterilizing action on bacteria except for spore-forming bacteria and suchlike that has high resistance when the ethanol concentration is equal to and higher than 60% by volume. This is a general view held by pharmacopoeias and others. However, when an additional experiment was conducted to ascertain whether or not a sterilizing effect can be produced only by humidifying the inside of the box with the chemical solution L1 with an ethanol concentration of 70% by volume, sterilization treatment against the spore-forming bacteria was not observed in this experiment.

As described above, it is observed that by the generation of ozone in an environment in which ethanol, an amphiphilic substance, is present, a high sterilizing effect on spore-forming bacteria can be produced.

### [Experiment 2]

According to the method described above, an experiment on sterilization treatment in a room was conducted to observe a sterilizing effect.

### The treatment space 1A subject to the experiment was one room with a size of 151 m³.

An object to be sterilized was a curtain provided in the room of the treatment space 1A. In this experiment, bacteria subject to sterilization treatment was Staphylococcus aureus.

The treatment space 1A was humidified with each of the chemical solution L1 (Example) in the embodiment and tap water (Reference Example 1).

A 10 *µ*L solution of Staphylococcus aureus with a concentration of 10⁸ CFU/mL was dropped on the curtain, and after being air-dried, the curtain was treated with ozone.

The sterilizing effect was evaluated by a method that includes checking numbers of the spore-forming bacteria adhering to the curtain before and after sterilization treatment, respectively, and a decrease in number.

### (Results)

Fig. 9 is a graph showing results of the experiment on sterilization treatment by ozone in Experiment 2. The vertical axis represents the number of surviving Staphylococcus aureus bacteria relative to an initial state on a logarithmic scale, and the horizontal axis represents the CT product [ppm·Min]. As shown in Fig. 9, until around 30 ppm·Min after a start of generation of the ozone, there has not been much difference in decreased number between Reference Example 1 and Example 1. However, the number sharply decreases at and after 60 ppm·Min in Example, showing a large difference in degree of decrease between the examples.

An estimated reason for the large difference is because adequate water has adhered to the surface of the Staphylococcus aureus when humidification is completed and generation of the ozone starts in Example, while it takes several tens of minutes for adequate water to adhere to the surface of the Staphylococcus aureus after the treatment space 1A is humidified and reaches predetermined humidity in Reference Example 1.

This confirms that Example produces a higher sterilizing effect in a short time compared to Reference Example 1.

### [Experiment 3]

According to the method described above, an experiment on sterilization treatment of a seed was conducted to observe a sterilizing effect.

An object to be sterilized was a bok choy seed. In this experiment, bacteria subject to sterilization treatment was Staphylococcus aureus.

The treatment space 1A was humidified with each of the chemical solution L1 (Example) in the embodiment and tap water (Reference Example 1).

The bok choy seed was immersed in a solution of Staphylococcus aureus with a concentration of 10⁷ CFU/mL and was air-dried for three hours after being taken out of the solution. After that, the seed was put in a glass box that has a volume of 100 L and was treated with ozone of 30 ppm.

The sterilizing effect was evaluated by a method that includes measuring numbers of the Staphylococcus aureus bacteria adhering to the seed before and after sterilization treatment, respectively, and checking a decrease in number.

### (Results)

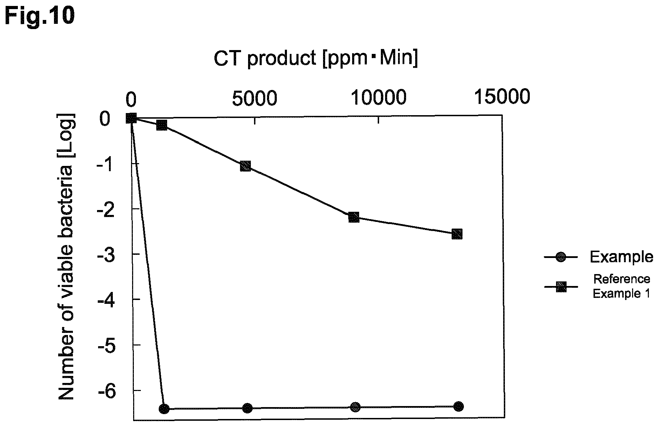

Fig. 10 is a graph showing results of the experiment on sterilization treatment by ozone in Experiment 3, and the vertical and horizontal axes are similar to those in Fig. 9 above. As shown in Fig. 10, while the bacteria number tends to gradually decrease after the start of ozone generation in Reference Example 1, there is a sharp decrease immediately after the generation of the ozone in Example.

An estimated reason for this is because adequate water has adhered to bacterial cells even despite presence of a fine structure such as downy hair of the seed when the bacteria is exposed to the ozone in Example, while the supply of moisture is hindered by the fine structure such as the downy hair of the seed in Reference Example 1.

This confirms that Example produces a higher sterilizing effect in a short time compared to Reference Example 1.

A CT value order of Experiment 2 was 100 ppm·Min, and a CT value order of Experiment 3 was 10000 ppm·Min. This confirms that even with a substantial change in CT value, Example produces a higher sterilizing effect in a short time.

### DESCRIPTION OF REFERENCE SIGNS

- 1: Box
- 1A: Treatment space
- 2: Door
- 3: Ventilating fan
- 20: Humidifier
- 21: Reservoir
- 22: Jet port
- 30: Ozone generator
- 31: Discharge lamp
- 32: Electrode
- 33: Gas inlet
- 33a: Air intake fan
- 34: Gas outlet
- 35: Flow path
- G1: Air
- G2: Ozone-containing gas
- L1: Chemical solution
- T1: Object to be treated
- X1: Ultraviolet light

## Claims

1. A sterilization treatment method that is a method for sterilization treatment by ozone in a treatment space defined, the sterilization treatment method comprising introducing ozone into the treatment space in an environment in which an amphiphilic substance is present.

2. The sterilization treatment method according to claim 1, further comprising generating ozone after spraying the amphiphilic substance diluted with water in the treatment space.

3. The sterilization treatment method according to claim 1, wherein the amphiphilic substance is a substance that does not have sterilizing action on spore-forming bacteria.

4. The sterilization treatment method according to claim 3, wherein the amphiphilic substance is alcohol or a surface-active agent.

5. The sterilization treatment method according to claim 4, wherein the amphiphilic substance is ethanol that is diluted with water such that a concentration of the ethanol ranges from 3% by volume to 60% by volume inclusive.

6. The sterilization treatment method according to claim 4, wherein the amphiphilic substance is polyoxyethylene sorbitan monooleate that is diluted with water such that a concentration of the polyoxyethylene sorbitan monooleate ranges from 0.5 g/L to 5 g/L inclusive.

7. The sterilization treatment method according to any one of claims 1 to 6, wherein the ozone introduced into the treatment space is generated by an ozone generator that includes a discharge lamp to emit ultraviolet light having a main emission wavelength in a range from 100 nm to 240 nm inclusive.

8. A sterilization treatment device comprising:
a humidifier that includes a reservoir to store an amphiphilic substance diluted with water, and a jet port to emit the amphiphilic substance diluted with the water in mist form, the amphiphilic substance flowing into the jet port from the reservoir; and
an ozone generator that includes a discharge lamp to emit ultraviolet light having a main emission wavelength in a range from 100 nm to 240 nm inclusive when an electrode is supplied with electric power, a gas inlet to take in air, and a gas outlet to discharge an ozone-containing gas that is generated when air taken in through the gas inlet is irradiated with the ultraviolet light from the discharge lamp.
